# EUROPEAN PATENT SPECIFICATION

(11) **EP 2 456 498 B1**
(45) Date of publication and mention of the grant of the patent: **29.05.2019**
(21) Application number: 10752184.1
(22) Date of filing: 19.07.2010
(51) Int. Cl.: A61M 25/00, A61M 25/10

(54) **A BALLOON FOR CATHETERS**
KATHETERBALLON
BALLONNET POUR CATHÉTERS

(30) Priority: 20.07.2009 IT TO20090544
(43) Date of publication of application: 30.05.2012
(73) Proprietor: Cid S.r.l., 13040 Saluggia (VC) (IT)
(72) Inventor: VALLANA, Franco, I-13040 Saluggia (VC) (IT); CURCIO, Maria, I-13040 Saluggia (VC) (IT); GASTALDIN, Davide, I-13040 Saluggia (VC) (IT)
(74) Representative: Bosotti, Luciano
(86) International application number: PCT/IB2010/053275
(87) International publication number: WO 2011/010273

(56) References cited:
- WO-A2-96/26748
- WO-A2-2006/114783
- US-A- 6 048 332
- US-A1- 2005 015 046
- US-A1- 2005 288 632

## Description

### Field of the invention

The present disclosure relates to balloons for catheters.

The present disclosure has been developed with particular attention paid to its possible application to angioplasty (with or without the use of stents) of blood vessels, such as for example the coronary arteries or peripheral vessels of various nature.

### Description of the prior art

The percutaneous transluminal angioplasty (PTA) techniques have estabilished themselves for years now in the treatment of stenotic vessels, such as for example the coronary arteries. In said context, there has been noted the importance of ensuring that the beneficial effects of the treatment last over time. For this reason, in addition to the treatments of pure and simple PTA, based upon a sequence of actions of dilation of the stenotic vessel performed with a balloon catheter, there has been extensive diffusion of techniques that envisage implantation, in the stenotic site, of a stent, i.e., of an expandable tubular structure that is able to produce a supporting action for the vessel wall in order to preserve in a durable way the conditions of perviousness achieved/restored with the angioplasty intervention. The implantation of stents does not, however, rule out the risk of restenosis phenomena of. This has led to the development of stents of the "drug-eluting" type that are able to release an active agent, such as for example an agent antagonistic to restenosis, on the site treated.

From documents such as, for example, US-A-5 232 444 (to which there corresponds the document No. WO-A-89/12478) there are on the other hand known balloon catheters that are able to deliver an active agent such as a drug thanks to the fact that the wall of the balloon is provided with openings (i.e., it is "porous"). Said openings allow to diffuse to the outside of the balloon a liquid introduced into the balloon itself and functioning as conveying liquid for the active agent. In particular, the liquid in question can be the pressurized liquid used for dilating the balloon on the site subjected to treatment. Solutions that are to some extent akin to these (which are applied also to localized-perfusion techniques) are known from various other documents such as, for example, WO-A-92/11895, WO-A-95/01200, WO-A-2009/036135, US-B-6 585 926, US-A-2003/0040712, US-A-2003/0158517, US-A-2003/0158517, US-A-2004/0260239, or JP-A-10337328, and, again, US-A-5 100 429 and US-A-5 213 576.

Further solutions are known from documents WO-A-2006/114783, US-A-2005/015046, US-A-6,048,332, US-A-2005/288632, WO-A-96/18427, and WO-A-96/26748. Amongst these, document WO-A-2006/114783 illustrates a solution in which the delivery of an active agent through the balloon occurs by means of pressurization of the balloon itself until a threshold pressure is exceeded, a condition that causes the opening of passageways for the active agent in pre-selected areas.

The solution proposed in document No. WO-A-2006/114783 envisages the use of preformed openings configured to reclose once the pressure inside the balloon drops below a preset value. Nonetheless, the above document suggests the use of leaflets applied to the openings and prearranged for operation as one-way valves.

However, the solutions proposed in document No. WO-A-2006/114783 present a series of drawbacks. First, no specific indication is given about possible geometries to obtain optimal conditions for closing the passageways that are created on the balloon. Second, the sole indication of a structural type that is provided envisages the application of leaflets in correspondence of the pre-formed openings, which proves substantially impracticable in view of the characteristics and of the dimensions typical of the structures to which reference is made. The typical diameter of the pre-formed openings that can be obtained with known manufacturing methods is in the vicinity of a few micrometres, at times just a little more than ten micrometres, which renders impracticable the installation of any accessory element, in view, above all, also of the non-negligible deformation that the balloon undergoes.

### Object and summary of the invention

The inventors have observed that, in diffusing via an expandable balloon an active agent such as an agent antagonistic to restenosis in the framework of an angioplasty treatment (with or without the application of a stent, possibly of the drug-eluting type), it is desirable to prevent as much as possible that:
- while the balloon is made to advance towards the implantation site, the active agent - which may be constituted, for example, by a cytostatic or cytotoxic agent - be dispersed in an undesirable way in the vascular system of the patient;
- when the balloon is cyclically "deflated" during the angioplasty treatment and detaches from the wall of the treated vessel, there be a penetration of blood within the balloon; and

- when, once the treatment is completed, the balloon is retracted outside the body of the patient, there be an undesirable dispersion of residues of active agent in the vascular system of the patient.

Furthermore, it is useful to be able to control the release of the active agent, causing - for example - the delivery of the active agent to intervene only after the surface of the dilated balloon, that has come into contact with the wall of the vessel, starts to perform a real action of dilation, for example with the effect of ablation of the sclerotic plaque.

The object of the present invention is to provide a balloon that is able to meet the requirements outlined above.

According to the present invention, said object is achieved thanks to a balloon having the features specifically recalled in the ensuing claims.

The description also provides a corresponding manufacturing method.

The claims form an integral part of the technical disclosure provided herein in relation to the invention.

### Brief description of the annexed drawings

The invention will now be described, purely by way of non-limiting example, with reference to the annexed drawings, wherein:
- Figure 1 is a general view of a balloon that can be used for angioplasty interventions;
- Figure 2 is a section according to the line II-II of Figure 1, reproduced at a modified scale;
- Figure 3 is a sectional view according to the line III-III of Figure 2, reproduced at a further enlarged scale;
- Figures 4 to 6 illustrate the behaviour of the part represented in Figure 3 during use of the balloon; and
- Figures 7 to 12 are schematic illustrations of successive steps of a method for producing a balloon for angioplasty treatments.

### Detailed description of embodiments

Illustrated in the ensuing description are various specific details aimed at an in-depth understanding of the embodiments. The embodiments may be obtained without one or more of the specific details, or with other methods, components, materials, etc. In other cases, known structures, materials, or operations are not illustrated or described in detail in order to avoid rendering the various aspects of the embodiments obscure.

Reference to "an embodiment" or "one embodiment" in the framework of this description is intended to indicate that a particular configuration, structure or characteristic described in relation to the embodiment is comprised in at least one embodiment. Hence, phrases such as "in an embodiment" or "in one embodiment" possibly present in various points of this description do not necessarily refer to one and the same embodiment. In addition, particular conformations, structures, or characteristics can be combined in any adequate way in one or more embodiments.

The references used herein are provided only for convenience and hence do not define the scope of protection or the scope of the embodiments.

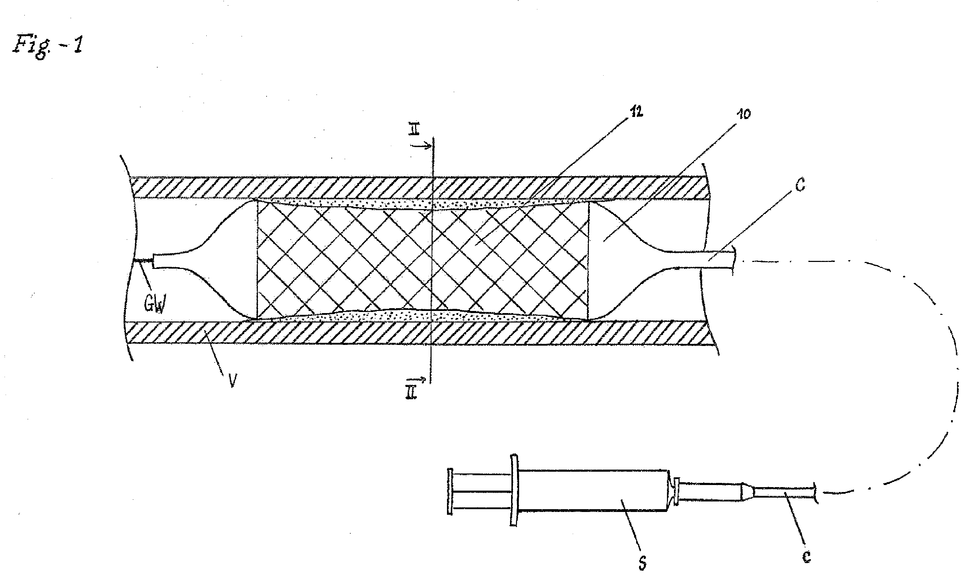

In Figure 1, the reference number 10 designates as a whole a balloon of a balloon catheter used for an angioplasty treatment (PTA) of a vessel V.

The balloon 10 is mounted at the distal end of an introduction catheter C intended to allow the introduction of the balloon 10 in a radially contracted condition, usually folded, into the body of the patient in view of its positioning, performed via catheterism (for example, resorting to a guide wire GW), in a site of a vessel V that is to be treated. The vessel in question may, for example, be one of the coronary arteries.

Once the balloon 10 has been placed in the implantation site, it may be dilated so as to assume the condition schematically represented in Figure 1 by supplying inside it an inflation fluid that is made to flow through the stem of the catheter C.

The fluid in question may be a liquid (for example, a physiological solution) pressurized by means of a syringe S actuated by an operator who introduces the pressurized liquid into the proximal end of the catheter C.

The techniques based upon the use of balloons of this type and the corresponding implementing details (including the possibility of using in conjunction with the balloon 10 a stent, possibly of the drug-eluting type) are to be deemed known in the art and hence such as not to require a detailed description herein. This applies also in relation to the choice of the materials constituting the balloon 10 and the ways in which the balloon itself is folded and mounted on the guide wire GW of the catheter used to obtain the advancement thereof towards the treated site.

In the embodiments considered herein, the balloon 10 is provided with an openwork or porous region, designated by 12, so as to enable an active agent (for example an agent antagonistic to restenosis) conveyed by the pressurized liquid that causes the expansion of the balloon 10 to be diffused in the wall of the vessel V.

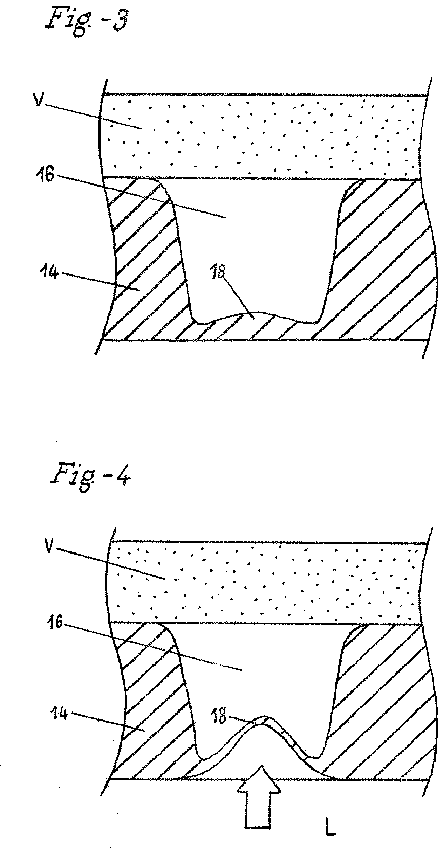

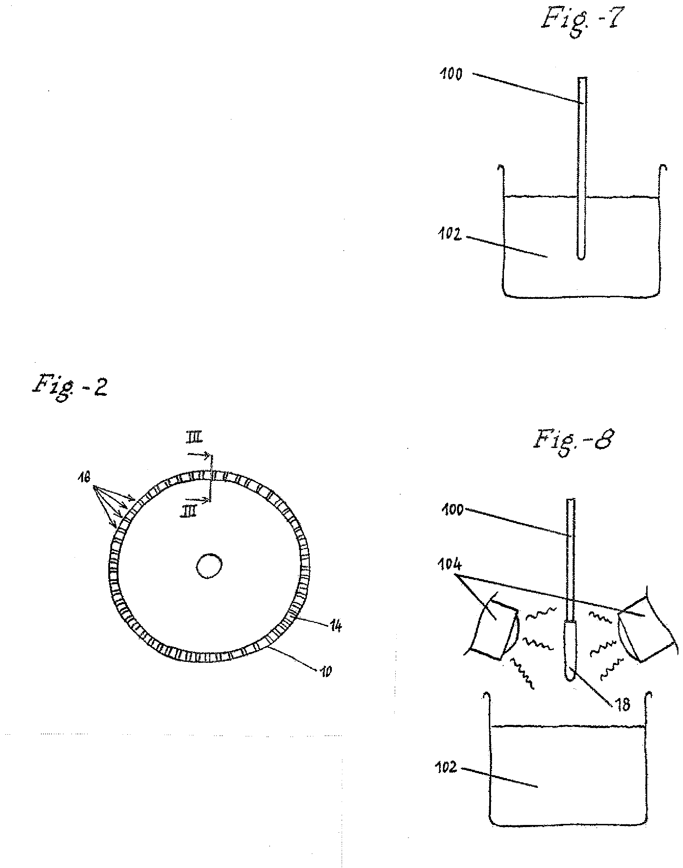

The cross-sectional view of Figure 2 highlights the fact that, in correspondence of the area 12, the wall 14 of the balloon 10 (here represented in an expanded condition) is provided with openings - i.e., passageways - 16, which have the characteristics that may be more readily appreciated with reference to Figures 3 to 6.

In all embodiments considered herein, the passageways 16 are basically configured as holes in which an obturator (in practice a septum) 18 is present, located for example in an end position, for instance at the radially internal end with respect to the development of the balloon 10. The inventive obturator 18 presents, also according to the manufacturing process, a shape at least slightly lenticular and plano-convex with the convexity facing the outside of the balloon 10.

As may be more readily appreciated in the view of Figure 3, when the balloon 10 is made to advance towards the implantation site, the holes 16 initially present in the form of blind holes.

The blind hole condition of the openings 16 is maintained when the inflation liquid that conveyes the active agent is initially made to flow within the balloon 10 and also during the initial step of expansion of the balloon 10, which is obtained at a pressure practically equal to the atmospheric pressure until the wall 14 of the balloon 10 comes into contact with the wall of the vessel V undergoing treatment (see, for example, the condition illustrated in Figure 1) .

In the above conditions, the fact that the openings or passageways 16 are blind holes prevents the active agent from being dispersed in an undesirable way.

After the wall 14 of the balloon 10 has come into contact with the wall of the vessel V, the vessel V itself starts to offer resistance to the further expansion of the balloon 10, and the pumping action of the syringe S (which may possibly be a motor-driven syringe) induces in the inflation fluid the increase in pressure that underlies the angioplasty treatment, which basically amounts to an action (or more correctly, to a repeated sequence of actions) of forced dilation of the vessel V.

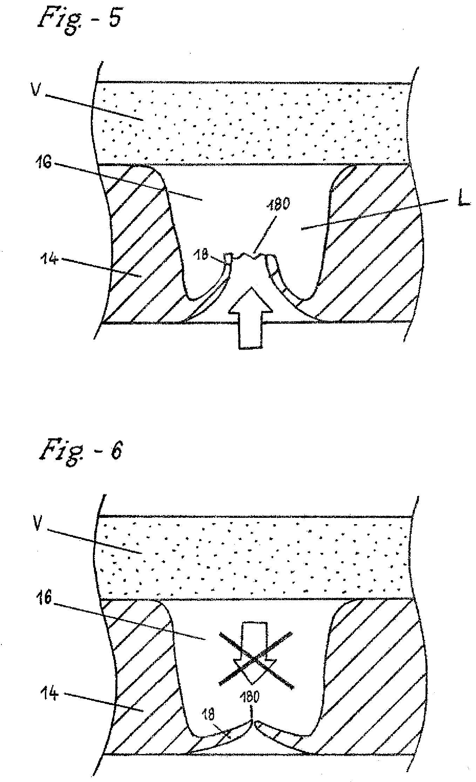

As may be appreciated from an observation of Figures 4 and 5 (where the relative proportions have been deliberately altered for reasons of clarity of illustration) the obturator 18 is intrinsically "thin" with respect to the overall thickness of the wall 14.

Just to clarify the idea -without this implying any limitation of the scope of the invention-, the wall 14 can have a thickness (measured in a radial direction with respect to the balloon 10) in the region of 10 to 50 µm-i.e., 10 to 50 x 10⁻⁶ metres - and the obturator 18 can have (also according to the materials used) a thickness from 10% to 50 % of the thickness of the wall 14.

The increase in pressure of the inflation liquid has the effect of deforming, i.e., bulging, the obturator 18 (in the embodiment illustrated, in which the obturator 18 is located at the internal end of the hole 16, there is an introflection within the hole itself) until the condition is reached in which, on account of its gradual bulging, the obturator forms a sort of bubble that explodes (Figure 5) creating an opening 180.

Just to clarify the idea (once again without this implying any limitation of the scope of the invention) the burst of the obturators 18 may occur at a pressure from 2 to 10 bar higher than the nominal operating pressure of the balloon 10, hence remaining in safety conditions below the rated burst pressure (RBP) of the balloon 10.

In this way, the hole 16 is rendered pervious, and the inflation liquid L is able to flow towards the wall of the vessel V so as to produce the desired action of diffusion of the active agent conveyed thereby.

From an observation of Figure 5 it may be noted that the active agent is practically all conveyed towards the wall of the vessel V. The formation of the opening 180 presupposes in fact that the wall 14 of the balloon 16 is forced in contact against the wall of the vessel V with a pressure equal to the pressure of the inflation liquid L.

This makes it possible to control the release of the active agent, causing delivery of the active agent only after the surface of the dilated balloon, that has come into contact with the vessel wall, starts to perform a real dilation action, for example with certain effect of ablation of the sclerotic plaque.

As already indicated previously, the angioplasty treatment can envisage an alternating sequence of expansions and contractions of the balloon 10 obtained by varying correspondingly (i.e., by increasing and decreasing) the pressure of the inflation liquid L.

The decrease in pressure of the inflation liquid leads to detachment of the wall of the balloon 14 from the wall of the vessel V: the action of deflation is in fact designed to enable flow of the blood within the treated vessel, which would be otherwise occluded by the balloon 10 when the balloon 10 itself is in the dilated condition.

As schematically illustrated in Figure 6, the modalities of formation of the opening 180, obtained by bursting a "bubble" created as a result of the deformation of the material forming the obturator 18 (which may be a material such as polyamide, e.g., Nylon 12) means that, when the pressure of the inflation liquid within the balloon 10 drops, the obturator 18 behaves as a whole more or less like the leaflets of a cardiac valve when the direction of the blood through the valve reverses with respect to the direction of free flow.

In other words, when the pressure of the inflation liquid drops, the obturator 18 tends to return approximately to the original condition so that the opening 180 is in effect choked (see once again Figure 6). The deformation of the material such as to lead to the formation of the "bubble", which then bursts giving rise to the opening 180, can in fact bring about a certain stretching of the material constituting the obturator.

Consequently, there is not any undesirable penetration of the blood in the balloon 10.

In this regard, it is also possible to intervene on the dimensions of the openings 16, causing them to give rise to phenomena of capillarity in regard to the inflation liquid and the active agent conveyed thereby. These phenomena can be useful to enable diffusion of the inflation liquid L (and of the active agent) towards the wall of the vessel V producing at the same time a trapping effect of possible air bubbles on the body of the balloon 10 - specifically within the openings 16 - preventing any undesirable phenomenon of diffusion of air in the blood flow, this being in any case a phenomenon of altogether minor importance, considering the dimensions of the objects involved.

For example, the holes 16 may have a diameter of less than 20 µm (20 x 10⁻⁶ metres) .

Also the choice of the material constituting the wall 14 (and hence the characteristics of wettability thereof) can come to play a role and be exploited in implementation of the mechanisms outlined above.

It will moreover be appreciated that when, once the treatment has been completed, the balloon 10 is finally deflated and then retracted out of the body of the patient, the drop in the pressure of the inflation liquid causes the obturators 18 to return into the original condition, with the openings 180 choked, thus preventing any undesirable dispersion of residue of active agent in the vascular system of the patient.

With reference to Figures 3 to 6 in which inventive obturators 18 are formed as convex frangible septa with the convexity facing the outside of the balloon 10. A septum which is convex towards the outside of the balloon 10 may exert a strong sealing action when the pressure of the inflation liquid within the balloon decreases, because possible flaps of the septum 18 created following upon burst thereof have a shape slightly arched outwards, which allows to reclose the aperture 180 with an action at least roughly assimilable to the action of the valve leaflets of a valve such as a heart valve.

A balloon 10 according to the embodiments considered herein can be obtained with various techniques.

For example, considering producing the balloon 10 with a polyamide-resin-based material, such as the material commercially known as Nylon 12, the wall 14 could be produced in the form of a double-layer structure with a thin internal tubular layer designed to constitute the obturators 18 fitted on which is a perforated tubular tunic, whose perforations or pores constitute the open part of the holes 16. The relative arrangement can even be reversed, with the thin layer set on the outside of the perforated tunic, in which case the obturators 18 are located at the outer end of the openings 16 (in Figures 3 to 6 the obturators 18 are instead located at the inner end). Also conceivable is recourse to a sandwich structure, with a thin layer designed to form the obturators 18 set between two perforated tunics, in which case the obturators 18 would be set in an intermediate position with respect to the development of the holes 16.

Figures 7 to 11 refer to an example of a method for producing a balloon 10 by resorting to a technique that can be substantially defined as a dipping technique, which can be used, for example, for producing balloons 10 with a base of a silicone material.

In Figures 7 to 11, the reference 100 designates a core (in practice a rod) having diametral dimensions that, in one example, substantially correspond to the desired diametral dimensions of the balloon 10 in an expanded condition.

The core 100 is dipped in a solution 102 of a precursor of the material constituting the balloon 10. When the core 100 is extracted from the solution 102, the precursor 102 forms a thin layer deposited on the wall of the core 100. In these conditions, the core 100 is exposed to the action of sources of lighting/heating 104 that bring about consolidation (i.e., curing) of the aforesaid precursor.

The sequence of operations represented in Figures 7 and 8 can be repeated a number of times according to the thickness desired for the layer of material corresponding to the obturators 18. According to the characteristics of the precursor 102 the greater the number of times the sequence of operations of Figures 7 and 8 is repeated, the thicker the obturators 18 will be. It will be appreciated that this procedure enables very precise adjustment of the thickness of the obturators 18 so as to ensure the desired operating modes.

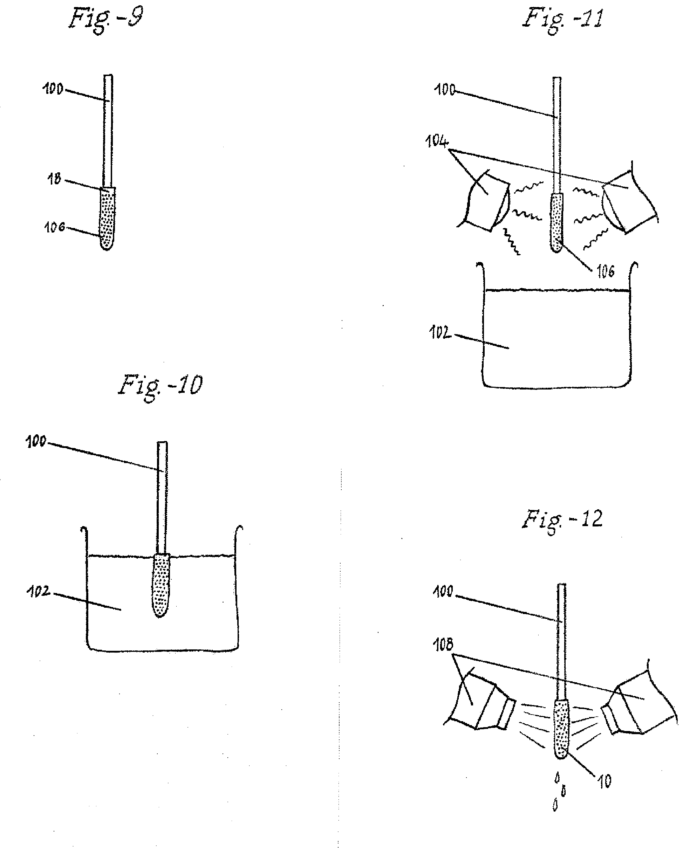

Figure 9 illustrates the possibility of depositing on the layer of material corresponding to the obturators 18 already formed on the core 100 a granular material 106 (such as, for example, a water-soluble granular material, for instance, granular glucose). Then (Figure 10), the operation of dipping of the core 100 in the precursor 102 is repeated (the precursor is here assumed as being of a nature identical to that of the solution of Figures 7 and 8, but it may also be of a different nature, so that at the end the obturators 18 are made of a material different from that of the rest of the wall portion 12).

Also in this case, the operation of extraction and exposure to the sources of lighting/heating 104 (Figure 11) aimed at promoting curing of the precursor can be repeated a number of times until, on the core 100, of a layer of material corresponding to the full parts of the wall 14 is formed. The areas occupied by the granules of the material 106 correspond, instead, to the openings 16.

As schematically illustrated in Figure 12, once the thickness of the wall 14 has reached the desired value, the core 100 with the material (cured) 106 deposited thereon is subjected to an operation - such as a washing operation, for example with water jets 108 - aimed at dissolving the material 106. In the position occupied by each of said granules, on the structure of balloon 10 formed on the core 100, an opening 16 will now be present having the characteristics illustrated in Figures 3 to 6.

It will be appreciated that the technique represented in Figures 7 to 12 is substantially equivalent to a technique commonly used for bestowing a granular surface appearance on silicone mammary prostheses.

Of course, without prejudice to the principle of the invention, the details of construction and the embodiments may vary, even significantly, with respect to what has been illustrated herein purely by way of non-limiting example, without thereby departing from the scope of the invention, as defined by the annexed claims.

## Claims

1. A balloon (10) for an angioplasty catheter expandable by means of a pressurized inflation liquid and including at least one wall portion (12) provided with passageways (16) for diffusing an active agent conveyed by said liquid, wherein the passageways (16) are provided with obturators (18) openable from an original blind hole condition of said passageways (16) under the pressure exerted by said pressurized liquid, wherein the obturators (18) are in the form of septa frangible under the pressure exerted by the pressurized liquid to form openings (180) for the agent conveyed by the pressurized liquid to flow through said passageways (16),
the balloon (10) being **characterized in that** in said original blind hole condition of said passageways (16) the obturators (18) are in the form of lenticular plano-convex septa with convexity facing outwardly of the balloon (10).

2. The balloon of claim 1, wherein the obturators (18) are closeable as the pressure of the pressurized liquid diminishes.

3. The balloon of any of the previous claims, wherein said wall portion (12) has a radially outer surface and a radially inner surface and wherein the obturators (18) are arranged at the radially inner surface.

4. The balloon of any of the previous claims, wherein the obturators (18) comprise portions of reduced thickness of said wall portion (12) of the balloon (10).

5. The balloon of any of the previous claims, wherein said wall portion (12) has a layered structure with a layer defining the obturators (18) and at least one perforated layer coupled to the layer defining the obturators (18).

6. The balloon of any of the previous claims, wherein:
- the obturators (18) are septa having a thickness between 10% and 50% of the thickness of said wall portion (12) of the balloon, and/or
- the passageways (16) have a diameter smaller than 20 micron.

## Patentansprüche

1. Ballon (10) für einen Angioplastie-Katheter, der mittels einer unter Druck stehenden Füllflüssigkeit aufdehnbar ist und mindestens einen Wandabschnitt (12) beinhaltet, der mit Durchgängen (16) zum Diffundieren eines durch die Flüssigkeit transportierten Wirkstoffs versehen ist, wobei die Durchgänge (16) mit Obturatoren (18) versehen sind, die aus einem ursprünglichen Sacklochzustand der Durchgänge (16) unter dem durch die unter Druck stehende Flüssigkeit ausgeübten Druck öffenbar sind, wobei die Obturatoren (18) in Form von Scheidewänden vorliegen, die unter dem durch die unter Druck stehende Flüssigkeit ausgeübten Druck aufbrechbar sind, um Öffnungen (180) für den durch die unter Druck stehende Flüssigkeit transportierten Wirkstoff zu bilden, um durch die Durchgänge (16) zu strömen,
wobei der Ballon (10) **dadurch gekennzeichnet ist, dass** in dem ursprünglichen Sacklochzustand der Durchgänge (16) die Obturatoren (18) in Form von linsenförmigen plankonvexen Scheidewänden mit einer zur Außenseite des Ballons (10) gewandten Konvexität vorliegen.

2. Ballon nach Anspruch 1, wobei die Obturatoren (18) bei abnehmendem Druck der unter Druck stehenden Flüssigkeit verschließbar sind.

3. Ballon nach einem der vorhergehenden Ansprüche, wobei der Wandabschnitt (12) eine radial außenliegende Oberfläche und eine radial innenliegende Oberfläche aufweist und wobei die Obturatoren (18) an der radial innenliegenden Oberfläche angeordnet sind.

4. Ballon nach einem der vorhergehenden Ansprüche, wobei die Obturatoren (18) eine verringerte Dicke aufweisende Abschnitte des Wandabschnitts (12) des Ballons (10) umfassen.

5. Ballon nach einem der vorhergehenden Ansprüche, wobei der Wandabschnitt (12) eine Schichtstruktur mit einer die Obturatoren (18) definierenden Schicht und mindestens einer mit der die Obturatoren (18) definierenden Schicht verbundenen perforierten Schicht aufweist.

6. Ballon nach einem der vorhergehenden Ansprüche, wobei:
- es sich bei den Obturatoren (18) um Trennwände handelt, die eine Dicke von zwischen 10 % und 50 % der Dicke des Wandabschnitts (12) des Ballons aufweisen, und/oder
- die Durchgänge (16) einen Durchmesser von weniger als 20 Mikrometern aufweisen.

## Revendications

1. Ballonnet (10) pour un cathéter d'angioplastie dilatable au moyen d'un liquide de gonflage sous pression et comportant au moins une partie de paroi (12) pourvue de passages (16) pour diffuser un agent actif transporté par ledit liquide, où les passages (16) sont pourvus d'obturateurs (18) pouvant s'ouvrir à partir d'un état de trou borgne d'origine desdits passages (16) sous la pression exercée par ledit liquide sous pression, où les obturateurs (18) se présentent sous la forme de cloisons frangibles sous la pression exercée par le liquide sous pression pour former des ouvertures (180) permettant à l'agent transporté par le liquide sous pression de s'écouler à travers lesdits passages (16),
le ballonnet (10) étant **caractérisé en ce que**, dans ledit état de trou borgne d'origine desdits passages (16), les obturateurs (18) se présentent sous la forme de cloisons plan-convexes lenticulaires dont la convexité est tournée vers l'extérieur du ballonnet (10).

2. Ballonnet de la revendication 1, dans lequel les obturateurs (18) peuvent être fermés lorsque la pression du liquide sous pression diminue.

3. Ballonnet de l'une des revendications précédentes, dans lequel ladite partie de paroi (12) présente une surface radialement extérieure et une surface radialement intérieure et dans lequel les obturateurs (18) sont agencés au niveau de la surface radialement intérieure.

4. Ballonnet de l'une des revendications précédentes, dans lequel les obturateurs (18) comprennent des parties d'épaisseur réduite de ladite partie de paroi (12) du ballonnet (10).

5. Ballonnet de l'une des revendications précédentes, dans lequel ladite partie de paroi (12) a une structure en couches avec une couche définissant les obturateurs (18) et au moins une couche perforée couplée à la couche définissant les obturateurs (18).

6. Ballonnet de l'une des revendications précédentes, dans lequel :
- les obturateurs (18) sont des cloisons ayant une épaisseur comprise entre 10% et 50% de l'épaisseur de ladite partie de paroi (12) du ballonnet, et/ou
- les passages (16) ont un diamètre inférieur à 20 microns.
